Europäisches Patentamt

European Patent Office

Office européen des brevets

(11) Publication number: **0 128 220**
A1

## EUROPEAN PATENT APPLICATION

published in accordance with Art. 158(3) EPC

(21) Application number: **83902573.1**

(22) Date of filing: **10.08.83**

Data of the international application taken as a basis:

(86) International application number: **PCT/JP 83/00256**

(87) International publication number: **WO 84/00680 (01.03.84 84/6)**

(51) Int. Cl.³: **A 61 F 1/03**

(30) Priority: **23.08.82 JP 144623/82**

(43) Date of publication of application: **19.12.84 Bulletin 84/51**

(84) Designated Contracting States: **CH DE FR GB LI**

(71) Applicant: **Limited Company YUSHIN KAIHATSU, 1285 Oaza Mizuhara Hirokawa-Cho Yame-Gun, Fukuoka-Ken 834-01 (JP)**

(72) Inventor: **HIMENO, Shinkichi, 13-29, Maidashi 4-chome Higashi-ku, Fukuoka-shi Fukuoka-ken 812 (JP)**

(74) Representative: **Daunton, Derek, Barlow, Gillett & Percival 94, Market Street, Manchester M1 1PJ (GB)**

(54) **LOW FRICTION ARTIFICIAL JOINT.**

(57) A low friction artificial joint with a liquid storage unit with ultrafine recesses formed by ultrafine holes or grooves 1–500 μm in width and 1–500 μm in depth formed over the sliding surface of a prosthetic material and a human joint, for holding a joint liquid.

ACTORUM AG

SPECIFICATION

## TITLE OF THE INVENTION

Low Friction Artificial Joint

## FIELD OF THE INVENTION

This invention is related to artificial joints which artificially replace human joints such as the hip joint and knee joint with prosthetic materials.

## BACKGROUND OF THE INVENTION

Human joints at all the hip, knees, shoulders, legs, elbows, hands and fingers require to be replaced with artificial joints in case of deformity or damage caused by illness, accident or deterioration by age.

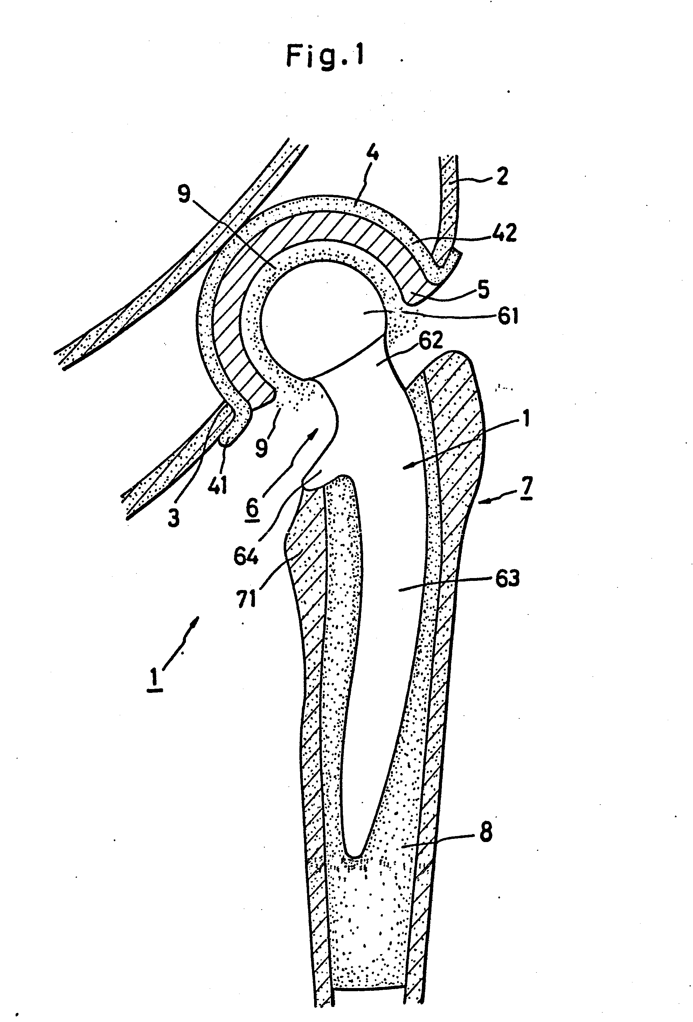

Fig. 1 is an explanatory drawing of replacing a hip joint with an artificial joint using prosthetic material and an acetabular tegmen socket. In the drawing, the artificial joint 1 which constitutes the prosthetic material is produced in the following procedure: The marrow of an acetabular tegmen 3 of the pelvis bone 2 is removed within a certain range, and a metal shell 4 made of a bowl 42 having a brim 41 is fitted into the surroundings of the inlet to the acetabular tegmen 3 and further a low-hardness bowl-shaped acetabular tegmen 5 made of polyethylene, etc., is fitted doubly into the metal shell 4. The artificial hip joint 6 consists of high-hardness materials such as

0128220

stainless steel, cobalt-chromium alloy, COP-1, ceramics, titanium alloy, etc. For stainless steel, SUS 316, SUS 316L, SUS 317, etc., are used, and COP-1 contains 20% cobalt, 20% chromium, 20% nickel, 4% molybdenum, and 0.2% phosphorous, Fe composing the rest. These artificial hip joints of high-hardness materials have the following construction: The joint rotatablly accommodates a smooth condyle 61, which may be of a semiglobular shape, semielliptic shape or other semicircular arc surfaces in the said acetabular tegmen socket 5. On the other hand, a stem part 63, which is set slenderly from the said condyle 61 through the head part 62, is inserted into a thigh 7, after the marrow in the thigh is removed, and the calcar connecting protrusion 64, which protrudes on the lower part of the said head part 62, is connected to the calcar part 71 which is the inlet part of the said thigh 7. Next, cement 8 is placed between the inside of the thigh 6 and a stem part 63, and the stem part 63 is fixed securely. Since the stem part 63 of the artificial hip joint 6 is firmly connected to the thigh 7 as explained above, the support of the pelvis bone 2 and thigh 7 is maintained by the sliding surfaces of the condyle 61 and the acetabular tegmen 5.

For primary human joint surface, there is an extremely viscous joint liquid with sufficient thickness between joint sliding surfaces, which slide on the liquid layer. Namely, they are in the fluid-lubricated condition. Therefore, the friction coefficient is within the range of as low as 0.01 to 0.001.

On the other hand, the artificial joint in the past was made so that both the surface of the condyle 61 and the inner cavity surface of the acetabular tegmen socket 5 would become smooth surfaces, and when sliding was forced under load during man's walking, etc., pressure pushing both the sliding surfaces against each other causes the lubricating liquid which has been placed between the sliding surfaces to be squeezed out through the joint gap. As a result, the lubricating oil film was broken and finally both the sliding surfaces came in contact with each other and wear was generated by physicochemical reaction. In fact the friction coefficient was in the range of 0.05 to 0.1 at the most which was higher value than that of the living body, and as a long time passes after the operation, the wear at soft acetabular socket 5 side also became heavily worn. Thus there were many examples that the mechanical play or irritation to the surrounding tissue by worn chips caused complaints of pain.

Namely, the manufacturers of artificial joints in the past were excessively swayed by their fixed idea that "the smoother the surface, the less the wear," and overlooked the aspect that the smoothly finished surface led to the breakage of lubricatin g oil film and increased the friction wear.

## SUMMARY OF THE INVENTION

The purpose of this invention is to eliminate such defects and supply low friction artificial joints, which permit the storage of the joint liquid by forming the liquid storing part consisting of minute concave parts on prosthetic material surfaces of the sliding surfaces between the two prosthtic surfaces or between the prosthetic material and the human body joint.

## BRIEF DESCRIPTION OF DRAWINGS

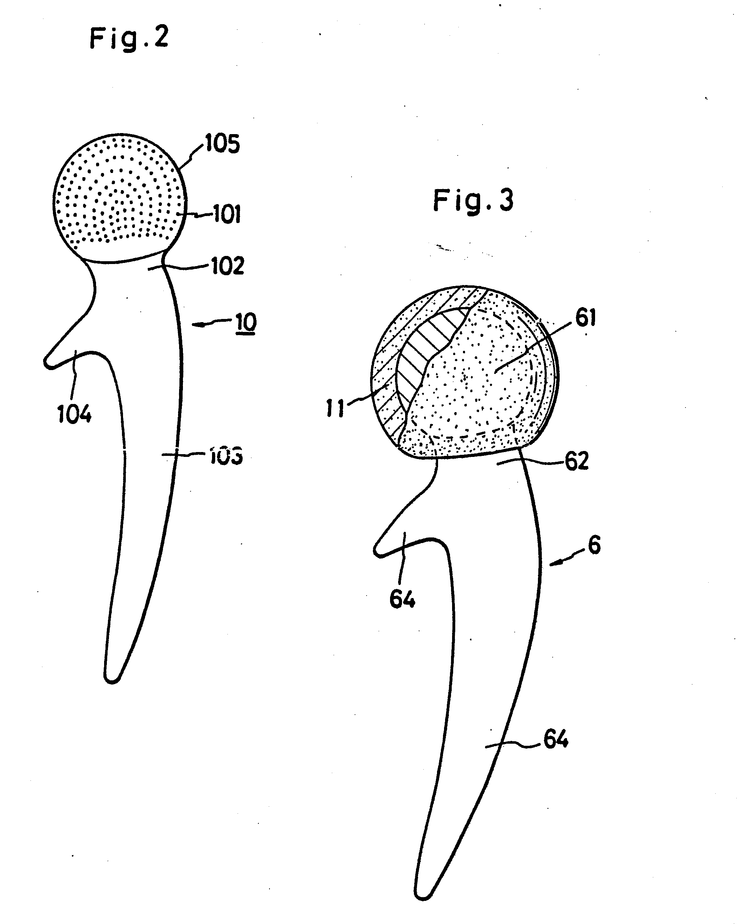

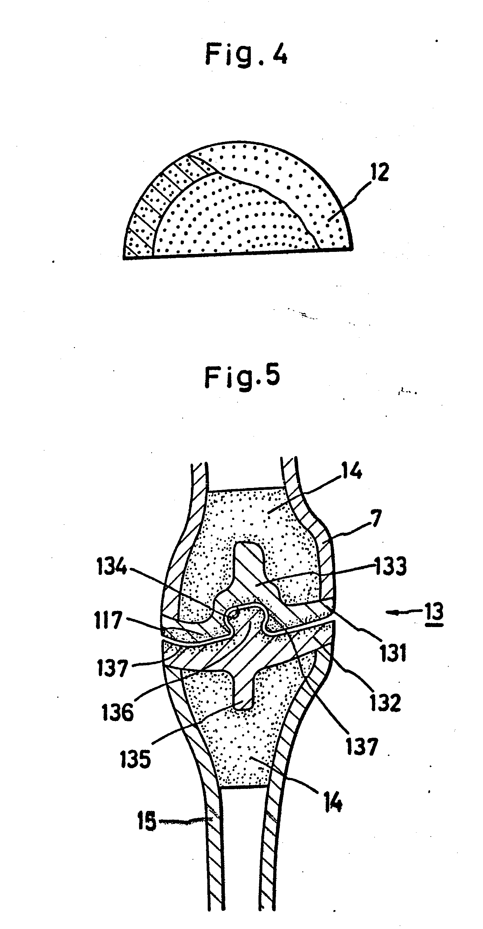

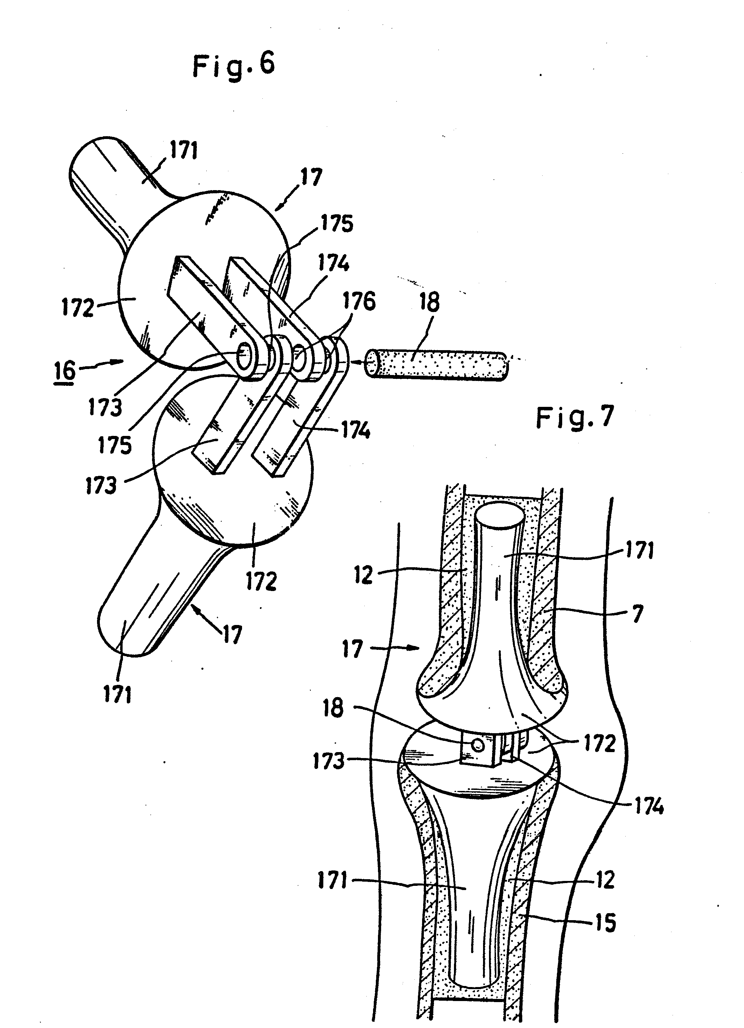

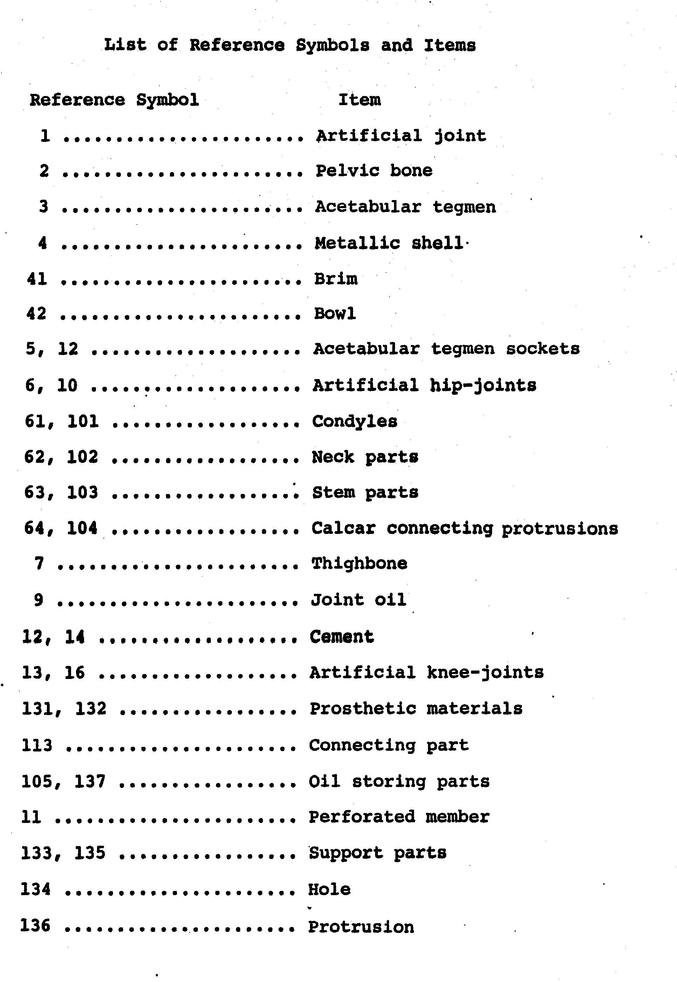

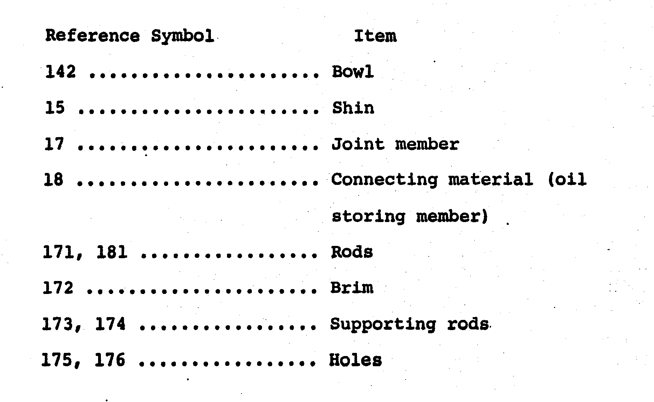

Fig. 1 is an explanatory drawing of the artificial joint, Fig. 2 is the side view of the artificial hip joint of this invention, Fig. 3 is the partially-cut-off side view of other artificial embodiment of the hip joint of the present invention, Fig. 4 is the side view of the partially-cut-off acetabular tegmen socket of the artificial hip joint used in this invention, Figs. 5 and 6 are the oblique views of other embodiments of the artificial knee joints of the present invention, and Fig. 7 is the explanatory drawing of the embodiment of Fig. 6 applied to a living body.

## DETAILED DESCRIPTION OF THE PREFERRED ENBODIMENTS

The preferred embodiments of the present invention are explained by referring to drawings, as follows:

Fig. 2 shows an oblique view of one embodiment of the present invention applied to the artificial hip joint as a prosthetic material. In the figure, the artificial hip joint 10 is formed by high-hardness materials such as stainless steel, cobalt-chromium alloy, COP-1 like the artificial hip joint in Fig. 1, and is equipped with a condyle 101, neck part 102, stem part 103, and calcar connecting protrusion 104. The neck part 102, stem part 103, and calcar connecting protrusion 104 correspond to the neck part 62, stem part 63, and calcar connecting protrusion 64 in Fig. 1 respectively. The condyle 101 forms a sliding surface having a semiglobular or semielliptic globular shape or other semiarc or swelling surface. It also has a joint oil storing part 105 which consists of minute concave parts for storing the joint oil. The oil storing part 105 in this preferred embodiment consists of minute holes of 1 to 500 microns in width and 1 to 500 microns in depth (the figure indicate them in enlarged forms for the sake of explanation). The existence of these minute holes contributes to the phenomenon that strong hydraulic pressure is generated

0128220

by the hydrodynamic effect on the minute holes and in the neighbour area at the time of sliding motion under load. This hydraulic pressure prevents sliding surfaces from approaching each other, and the lubricating oil film thickness can be kept to a sufficient level for fluid lubrication. The flowing-out of lubricating oil from the storing part to the surrounding area also stops the oil film breakage on the surface. To expect these effects, the storing part should have width in the range of 1 to 500 microns, and depth in the range of 1 to 500 microns. The storing part of 1 micron or below in both width and depth cannot store sufficient oil and the storing part of 500 microns or above in width and depth permits no sufficient hydrodynamics effect, due to very slow sliding speed of human body's joints. It cannot also be neglected that the curved sliding surface is hazardous, because the edge of the concave part will bit into the opposite sliding surface.

Here it is asuumed that the storing part is formed by photo-etching on the condyle metal surface, and it may be made by mechanical depression to produce traces or by mechanical scratching. Or the concave part can be formed mechanically into a slightly deeper level at the initial stage, and then the surface layer is ground

- 6 -

0128220

to cut off the piled-up portion and distorsion on the surface produced during machining for forming the concave parts, thereby finally forming a portion having design depth. Especially, when a ceramic condyle is used, the procedure of forming the deeper concave parts mechanically in its soft condition before sintering, and of grinding them after sintering is easy and benefitial. The oil storing part 105 is provided on the surface of the condyle in the above embodiment, but the condyle 101 may be formed with a perforated high-hardness material possessing minute holes inside, and the minute holes may be exposed on the sliding surface.

The surface shape of the oil storing part may be any shape of the circle, ellipse, polygon or irregular shape. The sectional shape of the oil storing part may be any of the arc, plate, or dish. It is preferable to shape the edge part into a smooth surface, because the smoother edge will minimize the wear. Even if the sliding surfaces approach each other, the wear can be minimized. This arrangement may be random or regular in a spiral or in a concentric circle.

Not only the minute holes can be set independently from one another, but also grooved concave parts may be formed at an oblique angle of 45° to the expected main sliding direction. In addition to the same benefit as the minute holes, this arrangement permits pushing lubricating oil along the grooves into the inner part of the sliding surface during sliding motion of the joint surface, and also generates force which acts in the direction of discharging the inner lubricating oil reversely in a relative motion between the sliding direction and the groove direction. This will contribute to supplying fresh joint oil to the sliding surface all the time and to mintaining excellent lubricating characteristics at all times.

Fig. 3 shows an artificial hip joint indicating another embodiment of this invention applied to the hip joint. In Fig. 3 the same numerical symbols as those in Fig. 1 indicate the same parts. The symbol 11 shows a low-hardness perforated material like polyethylene. The perforated material 11 has minute holes inside and covers the outer side of the condyle 61, thereby enabling it to store the joint oil on the sliding surface with the acetabular tegmen or its socket.

Fig. 4 is a partially-cut-off sectional view indicating a preferred embodiment of the artificial joint when the oil storing part is provided at the acetabular tegmen socket side. The acetabular tegmen socket 12 consists of a bowl of a semiglobular shape, and is made of low-hardness perforated material to form an oil storing part with exposed minute concaves.

The artificial joint in this preferred embodiment is used, replacing the acetabular tegmen socket 5 in Fig. 5 and also can be used in combination with the preferred embodiment in Fig. 2.

Fig. 5 explains a preferred embodiment of the artificial knee joint by applying the thigh and shin bone prosthetic materials to the knee joint. In Fig. 5, symbol 13 is an artificial knee joint forming the thigh prosthetic material, which consists of prosthetic materials 131 and 132. The prosthetic material 131 is equipped with a supporting part 133, which is inserted into the interior of the thigh 7, after its marrow is bored out, and is fixed with cement 14 at the reverse side. The prosthetic material is also equipped with a jar-shaped hole 134 at the center of the sliding surface on the front side. The prosthetic material 132 is equipped at its center with a supporting part 135 which is inserted by boring the shin bone 15 and is fixed with cement 14. A protrusion 136 on the sliding surface with the prosthetic material 131 at the front side is inserted into the hole 134 of the said prosthetic material 131, thereby making it possible to bend the thigh 7 and shin bone 15.

The oil storing part 137 is so formed that it has minute holes or grooves inside the jar-shaped hole 134 or on the sliding surface with other prosthetic material 132, thereby making it possible to store the joint oil.

If both prosthetic parts 131 and 132 in this preferred embodiment are made of high-hardness materials, a low-hardness perforated material can be included between them to form the oil storing part.

If one of the prosthetic materials 131 and 132 is made of a high-hardness material and the other is made of a low-hardness material, the oil storing part can be made on the surface of high-hardness material, or low-hardness material may be made of a perforated material and made into an oil storing part.

The oil storing part made of minute concave parts is formed on the surfaces of prosthetic materials 131 or 132, or the prosthetic materials 131 and 132 themselves may be made of a perforated material and the oil storing part may be exposed outside.

Fig. 6 is an oblique drawing of another preferred embodiment of the present invention, that is, an artificial knee joint using two prosthetic materials. Fig. 7 explains another preferred embodiment of applying the artificial knee joint in Fig. 6 to the knee of a living body.

In these drawings, the prosthetic knee joint 16 consists of a pair of joint materials 17 and the connecting material 18 (the oil storing material) which stores the oil in the minute concave parts on the surface of hard material, and also works as a hinge connection. The joint material 17 is made of high-hardness material the same as the material of the artificial joint 6, and consists of the following: The rod 171, which is made of hard material and inserted into the bone after the boring-out of the marrow, brim 172 connected with this rod 171 and shaped to increase its diameter gradually into a brim-like part, supporting rods 173 and 174 which protrude in parallel with the smooth surface of the brim 172, and holes 175 and 176 drilled into the tips of these supporting rods 173 and 174. The connecting material 18 is inserted into the holes 175 and 176 in combination to form a hinge-connection, thereby producing a sliding surface. This connecting material 18 has minute concave parts each having a diameter of 100 microns inside or at least on the surface of it.

Consequently, the connecting material 18 is inserted in the arrow direction into the holes 175 and 176 of paired joint materials 17 in Fig. 6 and is pushed out through the outsides of the outer holes 175 and 176, and this protruded part has its diameter increased and is caulked, thereby preventing it form slipping off.

Fig. 6, the connecting material 18, which forms the oil storing part, is equipped with minute concave parts on its hard material surface, but a perforated hard material also may be used to expose the minute holes on its surface.

The oil storing part may be formed by providing minute concave parts on the sliding surfaces of supporting rods 173 and 174, or may be formed by making the supporting rods with perforated material and by causing minute concave parts to be exposed on the surface.

Fig. 7 shows the preferred embodiment of the present invention applied to the living body's knee, and the same numerical symbols indicate the same materials.

Marrows in the thigh 7 and shin bone 15 are bored, and the rods 171 are inserted into them respectively, and fixed with cement 12. The connecting material 18 is included in the hinge coupling part. Therefore, even if the respective supporting rods are made of high-hardness materials, the joint oil is stored sufficiently and lubricating oil film is maintained at a desired level.

As mentioned above, the use of the artificial joint of the present invention permits the following: In connecting two prosthetic materials, it is sufficient to include an oil storing part, which has low hardness and minute holes, between the connecting sliding surfaces.

For sliding surfaces which are connected by the prosthetic material and the acetabular tegmen or by the prosthetic material and the acetabular tegmen socket, it is sufficient to form an oil storing part, which has minute concave parts, on the prosthetic material, acetabular tegmen, or surfaces thereof, in order that the joint oil will always be maintained on the sliding surfaces without being squeezed out. As a result, sliding surfaces move on the included oil film layer, and a low-friction sliding motion, which resembles that by live joints, becomes possible under the load 3 or 5 times heavier than the human body weight. In addition, the present invention has the following merits: The sliding surfaces make no direct contact to each other, unlike the artificial joints in the past, thereby practically eliminating wear and greatly extending the life of the artificial joints.

CLAIMS

(1) A low-friction artificial joint wherein the storage of joint oil is made possible by forming an oil storing part composed of minute concave parts on the prosthetic material surfaces of sliding surfaces to be formed by two prosthetic materials or by a prosthetic material and a living joint.

(2) Teh low-friction artificial joint of Claim 1 featuring that the oil storing part is formed on the sliding surface of a prosthetic material.

(3) The low-friction artificial joint of Claim 1 featuring that the oil storing part is formed by exposing a perforated prosthetic material on the sliding surface.

(4) The low-friction artificial joint of Claim 1 featuring that one of the sliding surfaces which are formed by two prosthetic materials is formed by a high-hardness material and the other is formed by a perforated material of low hardness.

(5) The low-friction artificial joint of Claim 1 featuring that the oil storing part is formed by minute holes or grooves each having a width of 1 micron to 500 microns and a depth of 1 micron to 500 microns.

(6) The low-friction artificial joint of Claim 1 featuring that sliding surfaces, which are formed by prosthetic materials are of a hinge connection and fine concave parts are provided on the sliding surfaces.

Fig.1

2/6

0128220

Fig.2

105
101
102
10
104
103

Fig.3

61
11
62
6
64
64

# Fig. 4

# Fig. 5

4/6.   0128220

Fig.6

Fig.7

## List of Reference Symbols and Items

| Reference Symbol | Item |
|---|---|
| 1 | Artificial joint |
| 2 | Pelvic bone |
| 3 | Acetabular tegmen |
| 4 | Metallic shell |
| 41 | Brim |
| 42 | Bowl |
| 5, 12 | Acetabular tegmen sockets |
| 6, 10 | Artificial hip-joints |
| 61, 101 | Condyles |
| 62, 102 | Neck parts |
| 63, 103 | Stem parts |
| 64, 104 | Calcar connecting protrusions |
| 7 | Thighbone |
| 9 | Joint oil |
| 12, 14 | Cement |
| 13, 16 | Artificial knee-joints |
| 131, 132 | Prosthetic materials |
| 113 | Connecting part |
| 105, 137 | Oil storing parts |
| 11 | Perforated member |
| 133, 135 | Support parts |
| 134 | Hole |
| 136 | Protrusion |

6/6          0128220

| Reference Symbol | Item |
| --- | --- |
| 142 ........................ | Bowl |
| 15 ......................... | Shin |
| 17 ......................... | Joint member |
| 18 ......................... | Connecting material (oil storing member) |
| 171, 181 ................... | Rods |
| 172 ........................ | Brim |
| 173, 174 ................... | Supporting rods |
| 175, 176 ................... | Holes |

# INTERNATIONAL SEARCH REPORT

0128220

International Application No. PCT/JP83/00256

## I. CLASSIFICATION OF SUBJECT MATTER (if several classification symbols apply, indicate all) [3]

According to International Patent Classification (IPC) or to both National Classification and IPC

Int. Cl.$^3$ A61F 1/03

## II. FIELDS SEARCHED

### Minimum Documentation Searched [4]

| Classification System | Classification Symbols |
|---|---|
| I P C | A61F 1/03, 1/00 |

### Documentation Searched other than Minimum Documentation to the Extent that such Documents are Included in the Fields Searched [5]

| | | |
|---|---|---|
| | Jitsuyo Shinan Koho | 1926 – 1982 |
| | Kokai Jitsuyo Shinan Koho | 1971 – 1982 |

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| X | JP,A, 52-2092 (Gebrüder Sulzer A.G.), 8. January. 1977 (08. 01. 77) & DE,A, 2527865 & US,A, 4031570 | 1, 2 |
| X | JP,A, 50-55194 (Oskowal A.G.), 15. May. 1975 (15. 05. 75) & US,A, 3894297 | 1, 2 |
| X | JP,A, 51-148290 (Gebrüder Sulzer A.G.), 20. December. 1976 (20. 12. 76) & US,A, 4032994 | 1, 2 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| October 20, 1983 (20. 10. 83) | October 31, 1983 (31. 10. 83) |
| International Searching Authority [1] Japanese Patent Office | Signature of Authorized Officer [20] |

Form PCT/ISA/210 (second sheet) (October 1981)